# EUROPÄISCHE PATENTANMELDUNG

(11) **EP 4 763 151 A1**
(43) Veröffentlichungstag der Anmeldung: **24.06.2026**
(21) Anmeldenummer: 25226995.6
(22) Anmeldetag: 23.12.2025
(51) Int. Cl.: A61F 5/01, A61F 5/30

(54) **ELLENBOGENSPANGE, PELOTTE**

(30) Priorität: 23.12.2024 DE 102024139647
(71) Anmelder: Sporlastic GmbH & Co. KG, 72622 Nürtingen (DE)
(72) Erfinder: Laitzsch, Lukas, 10829 Berlin (DE); Horst, Christian, 73230 Kirchheim u. Teck (DE); Blanc, Stephan, 8404 Winterthur (CH); Schmeltzpfenning, Timo, 72074 Tübingen (DE)
(74) Vertreter: DREISS Patentanwälte PartG mbB

(57) **Zusammenfassung**

Die Erfindung betrifft eine Ellenbogenspange (10) zur Entlastung einer radialen und/oder ulnaren Unterarmmuskulatur (12, 14) eines Patienten, umfassend wenigstens ein Schalenelement (16) zur medialen und/oder lateralen Anordnung an den Unterarm des Patienten; wenigstens ein Bandelement (18) zum Befestigen des wenigstens einen Schalenelements (16) an dem Unterarm des Patienten; wenigstens eine am wenigstens einen Schalenelement (16) definiert angeordnete Pelotte (42) mit wenigstens einer sich entlang einer Rippenachse (52) erstreckenden Rippe (54), wobei die Pelotte (42) in einer definierten ersten Konfiguration, in welcher die Pelotte (42) an der radialen Unterarmmuskulatur (12) anordenbar ist, und einer definierten zweiten Konfiguration, in welcher die Pelotte (42) an der ulnaren Unterarmmuskulatur (14) anordenbar ist, einrichtbar ist.

## Beschreibung

Die Erfindung betrifft eine Ellenbogenspange, auch Epicondylitis-Spange genannt, mit einer lösbar oder nicht lösbaren Pelotte sowie eine Pelotte zur Anordnung an die Ellenbogenspange.

Aus der EP 2 977 031 ist eine Ellenbogenspange mit zwei Pelotten bekannt, wobei die Pelotten mittels eines Klettverschlusses an einem gebogenen Formteil angeordnet werden.

Der Erfindung liegt die Aufgabe zugrunde, eine Ellenbogenspange welche den Nachteilen des Standes der Technik begegnet, insbesondere die Druckeinwirkung auf die Unterarmmuskulatur des Patienten verbessert und eine hohe Flexibilität bei der Behandlung der radialen und ulnaren Unterarmmuskulatur eines Patienten aufweist.

Die der Erfindung zugrundeliegende Aufgabe wird gelöst durch eine Ellenbogenspange mit den Merkmalen des Anspruchs 1. Die Erfindung ist gerichtet auf eine Ellenbogenspange zur Entlastung einer radialen und/oder ulnaren Unterarmmuskulatur eines Patienten, umfassend: wenigstens ein Schalenelement zur medialen und/oder lateralen Anordnung an den Unterarm des Patienten; wenigstens ein Bandelement zum Befestigen des wenigstens einen Schalenelements an dem Unterarm des Patienten; wenigstens eine am wenigstens einen Schalenelement definiert angeordnete Pelotte mit wenigstens einer sich entlang einer Rippenachse erstreckenden Rippe. Die Pelotte ist in einer definierten ersten Konfiguration und einer definierten zweiten Konfiguration einrichtbar. In der definierten ersten Konfiguration kann mittels der Pelotte die radiale Unterarmmuskulatur behandelt werden ("Tennisarm"). In der definierten zweiten Konfiguration kann mittels der Pelotte die ulnare Unterarmmuskulatur behandelt werden ("Golferarm").

Im Sinne der Erfindung ist unter einer "definierten Konfiguration" zu verstehen, dass die Pelotte in einer wiederholgenauen räumlichen Lage relativ zur Unterarmmuskulatur im Tragezustand angeordnet ist. Wenn die Änderung der Konfiguration der Pelotte mittels der Änderung der Position und/oder Orientierung der Pelotte am Schalenelement erzielt wird, ist unter der "definierten Konfiguration" zu verstehen, dass die Pelotte in einer wiederholgenauen räumlichen Lage relativ zum Schalenelement angeordnet ist. Wenn die Änderung der Konfiguration der Pelotte mittels der Änderung der Position und/oder Orientierung des Schalenelements erzielt wird, ist unter der "definierten Konfiguration" zu verstehen, dass die Pelotte in einer wiederholgenauen räumlichen Lage relativ zu einer Unterarmaufnahme und/oder zur Unterarmmuskulatur angeordnet ist.

Indem die Pelotte in zwei Konfigurationen eingerichtet werden kann, kann die Ellenbogenspange flexibel zur Behandlung der radialen und der ulnaren Unterarmmuskulatur verwendet werden, wobei dabei das wenigstens eine Schalenelement und die wenigstens eine Pelotte zum Einsatz kommen können. Demnach kann beispielsweise mit einer Pelotte sowohl die radiale als auch die ulnare Unterarmmuskulatur behandelt werden. Die Pelotte kann zum einen entweder dorsal oder versal und/oder zum anderen entweder an der radialen oder an ulnaren Unterarmmuskulatur eingesetzt werden. Folglich ist die Pelotte besonders flexibel einsetzbar. Somit kann eine Behandlung eines Tennisarms oder eines Golferarms mit der Ellenbogenspange behandelt werden.

Im Sinne der Erfindung ist unter einer "Pelotte" ein Stützkörper zu verstehen, welcher eine Massagewirkung auf die anliegende Muskulatur ausübt. Dabei wird das unter der Pelotte liegende Muskelgewebe einer, insbesondere temporal variablen, Schub- oder Spannungsbeaufschlagung ausgesetzt. Unter einer Pelotte sind im Sinne der Erfindung nur derartige Stützkörper zu verstehen, die mittels einer, insbesondere umschlingenden, also Körper bzw. Körperteil umschließenden, Bandage am Körper bzw. einem Körperteil des Patienten befestigt werden, insbesondere an diesen angepresst werden. Es sind keine Massagematten oder gar Einlegesohlen für Schuhe unter einer Pelotte im vorliegenden Sinne zu verstehen. Diese werden nicht mittels einer Bandage am Körper bzw. einem Körperteil des Trägers befestigt, sondern wirken über das Körpergewicht des Patienten. Der erfindungsgemäße Stützkörper hingegen wirkt vorzugsweise über eine Druckbeaufschlagung, die mittels der o.g. Bandage realisiert wird, stützend und massierend auf den Körper des Patienten. Insbesondere werden Stützkörper im vorliegenden Sinne muskel- und/oder gelenksnah angebracht.

Die Pelotte ist vorzugsweise zumindest im Wesentlichen kreuzförmig ausgebildet.

Ein vorteilhafter Aspekt der Erfindung sieht vor, dass die wenigstens eine Pelotte wenigstens zwei oder drei sich parallel zur Rippenachse erstreckende Rippen, aufweist. Demnach kann eine besonders effektive und zielgerichtete Massagewirkung der Unterarmmuskulatur erzielt werden.

Ein vorteilhafter Aspekt der Erfindung sieht vor, dass das wenigstens eine Schalenelement und die wenigstens eine Pelotte lösbar oder unlösbar miteinander verbunden sind. Aufgrund der Lösbarkeit der Pelotte kann diese individuell auf den Patienten angepasst angeordnet werden. Die Pelotte ist ferner so einfach austauschbar.

Ein vorteilhafter Aspekt der Erfindung sieht vor, dass das wenigstens eine Schalenelement wenigstens eine Ausnehmung und/oder die wenigstens eine Pelotte wenigstens einen Befestigungsabschnitt zum formschlüssigen und lösbaren Verbinden des wenigstens einen Schalenelements und der wenigstens einen Pelotte aufweist. Demnach kann die Pelotte zum Ändern der Konfiguration vom Schalenelement gelöst werden. Alternativ kann zum Ändern der Konfiguration der Pelotte das Schalenelement gedreht werden. Vorzugsweise ist dazu das wenigstens eine Schalenelement lösbar von dem wenigstens einen Bandelement ausgebildet.

Ein vorteilhafter Aspekt der Erfindung sieht vor, dass die wenigstens Pelotte senkrecht zur Rippenachse asymmetrisch ausgebildet ist. Dies kann z.B. dadurch erzielt werden, dass die wenigstens eine Rippe außermittig gegenüber einer senkrecht zur Rippenachse verlaufenden Quererstreckung der wenigstens einen Rippe ausgebildet ist. Die Quererstreckung ist durch die beiden freien Enden der Pelotte definiert, welche sich senkrecht zur Rippenachse von der Rippe wegerstrecken. Demnach kann eine gezielte Druckeinwirkung auf die Unterarmmuskulatur, insbesondere am Muskelansatz, erzielt werden.

Ein vorteilhafter Aspekt der Erfindung sieht vor, dass die wenigstens eine Pelotte in der ersten Konfiguration in einer ersten Pelottenorientierung am wenigstens einen Schalenelement angeordnet ist, wobei die wenigstens eine Pelotte in der zweiten Konfiguration in einer zweiten Pelottenorientierung am wenigstens einen Schalenelement angeordnet ist, und wobei die erste Pelottenorientierung und die zweite Pelottenorientierung invertiert zueinander ausgebildet sind. Demnach kann durch eine Drehung der Pelotte mit dieser sowohl die radiale als auch die ulnare Unterarmmuskulatur behandelt werden. Im Sinne der Erfindung ist unter einer "Invertierung" zu verstehen, dass die Pelotte von der ersten Pelottenorientierung in die zweite Pelottenorientierung um wenigstens 90°, insbesondere um wenigstens 135°, vorzugsweise um wenigstens 160°, bevorzugt um wenigstens 170°, gedreht ist. Vorzugsweise ist die Ausrichtung der Pelotte in beiden Konfigurationen um 180° gedreht (invertiert).

Ein vorteilhafter Aspekt der Erfindung sieht vor, dass das wenigstens eine Schalenelement in der ersten Konfiguration der wenigstens einen Pelotte in einer ersten Schalenorientierung am wenigstens einen Bandelement und/oder am Unterarm des Patienten angeordnet ist, wobei das wenigstens eine Schalenelement in der zweiten Konfiguration der wenigstens einen Pelotte in einer zweiten Schalenorientierung am wenigstens einen Bandelement und/oder am Unterarm des Patienten angeordnet ist, und wobei die erste Schalenorientierung und die zweite Schalenorientierung invertiert zueinander ausgebildet sind. Demnach kann durch eine Drehung des Schalenelements mit der Pelotte sowohl die radiale als auch die ulnare Unterarmmuskulatur behandelt werden.

Ein vorteilhafter Aspekt der Erfindung sieht vor, dass die Ellenbogenspange ein erstes Schalenelement und ein zweites Schalenelement aufweist. Das erste Schalenelement und das zweite Schalenelement sind vorzugsweise separat und/oder identisch zueinander ausgebildet. Die Schalenelemente sind vorzugsweise an zwei gegenüberliegenden Enden über jeweils zwei Bandelemente, insbesondere Gurte, miteinander verbunden, wobei insbesondere ein erstes Bandelement zur Festlegung des Abstands der Schalenelemente zueinander und/oder das zweite Bandelement zum Anlegen der Ellenbogenspange an dem Unterarm dient. In der ersten Konfiguration ist die Pelotte zur Behandlung der radialen Unterarmmuskulatur ("Tennisarm") vorzugsweise an dem ersten Schalenelement, insbesondere in der ersten Pelottenorientierung, anordenbar oder angeordnet. In der zweiten Konfiguration ist die Pelotte zur Behandlung der ulnaren Unterarmmuskulatur ("Golferarm") vorzugsweise an dem zweiten Schalenelement, insbesondere in der zweiten Pelottenorientierung, anordenbar oder angeordnet ist. Vorzugsweise sind beide Schalenelemente in einer ersten Schalenorientierung oder in einer zweiten Schalenorientierung, also in derselben Ausrichtung, angeordnet. Vorzugsweise ist dabei die Orientierung bzw. Ausrichtung der Pelotte in beiden Konfigurationen um 180° gedreht (invertiert).

Ein vorteilhafter Aspekt der Erfindung sieht vor, dass wenigstens eine erste Ausnehmung und wenigstens eine zweite Ausnehmung im wenigstens einen Schalenelement vorgesehen sind, und wobei die wenigstens eine Pelotte in der ersten Konfiguration an der wenigstens einen ersten Ausnehmung und in der zweiten Konfiguration an der wenigstens einen zweiten Ausnehmung angeordnet ist.

Ein vorteilhafter Aspekt der Erfindung sieht vor, dass das wenigstens eine Schalenelement eine Wabenstruktur aufweist. Vorzugsweise ist die wenigstens eine Ausnehmung Teil der Wabenstruktur. Aufgrund der Kombinationen aus formgebender Wabenstruktur, formschlaffen Bandelement und lokal wirkender Pelotte kann gezielt die Unterarmmuskulatur eines Patienten massiert werden, sodass Verspannungen durch das Tragen der Ellenbogenspange gelöst werden können. Aufgrund der Wabenstruktur ist eine besonders leichte, gezielt stabilisierend und anpassungsfähige sowie ästhetische Ellenbogenspange bereitgestellt. Aufgrund der Ausnehmung in der Wabenstruktur kann die lösbare Pelotte einfach an der Wabenstruktur befestigt werden. Ein zusätzliches Befestigungsmittel ist für die Pelotte an der strukturgebenden Wabenstruktur nicht erforderlich. Eine Ellenbogenspange hat gegenüber einem textilen Band den Vorteil, dass die Blutzirkulation nicht abgeschnürt und der Radial- bzw. Ulnarnerv nicht durch den Druck im Querschnitt eingeengt wird.

Die Ellenbogenspange ist vorzugsweise in der Spangenweite bzw. dorsal einstellbar, insbesondere mittel des wenigstens einen Bandelements. Demnach kann die Ellenbogenspange auf den Armumfang eingestellt werden und die durch die Pelotte bewirkte Position und Richtung der Druckkraft eingestellt werden. Eine in der Weite einstellbare Ellenbogenspange bewirkt, dass die Druckerhöhung nur von sagittaler Richtung wirkt. In der Ellenbogenbeuge und im dorsalen Bereich der Ellenbogenspitze sind Druckkräfte reduziert, sodass Einengungen ausgeschlossen werden. Ferner kann mittels der Weiteneinstellung auch die Position der Pelotte bestimmt werden.

Im Sinne der Erfindung ist unter einer "Wabenstruktur" eine stabilitätsgebende Strukturkomponente zu verstehen, welche über ihre gesamte flächige Erstreckung eine unregelmäßige Wabenstruktur aufweist. Die unregelmäßige Wabenstruktur weist vorzugsweise Stege aus festigkeitsgebendem Strukturmaterial auf und die Stege begrenzen unregelmäßig geformte strukturmaterialfreie Aussparungen. In stabilisierenden Bereichen können die Stege dicker ausgeführt sein, als in anpassbaren Bereichen. Die Wabenstruktur ist vorzugsweise flächig ausgebildet. Mit flächig ist dabei eine Ausgestaltung der Wabenstruktur gemeint, in der die Stärke der Wabenstruktur in Richtung der lokalen Flächennormalen deutlich geringer ist, als die Dimensionierung der Wabenstruktur entlang ihrer flächigen Erstreckung. Mit einer flächigen Erstreckung ist dabei jedoch nicht die Erstreckung in einer geometrischen Ebene gemeint, sondern die Wabenstruktur kann eine dreidimensional geschwungene bzw. gekrümmte Form aufweisen. Typischerweise ist die Wabenstruktur komplementär zu der zu unterstützenden Gliedmaße bzw. der Körperform in einer Gelenksumgebung ausgebildet.

Die Wabenstruktur kann wenigstens eine in der flächigen Erstreckung liegende Stabilisierungsrichtung aufweisen. Quer zur Stabilisierungsrichtung ist die Wabenstruktur lokal (in einem Bereich der Wabenstruktur) stabilisierend biegestarr ausgebildet. Im angelegten Zustand der Orthese ist die Wabenstruktur derart angeordnet, dass die Stabilisierungsrichtung der Wabenstruktur entlang des Körperteils, insbesondere des Unterarms des Patienten verläuft, so dass das zu stabilisierende Körperteil des Patienten durch die Wabenstruktur stabilisiert wird. Eine Biegung quer zur Stabilisierungsrichtung ist nur minimal möglich. Quer zur Stabilisierungsrichtung ist die Wabenstruktur also derartig starr ausgebildet, dass sie zur Stütze bzw. Stabilisierung einer Gliedmaße des Patienten der Orthese dient (im angelegten Zustand). Mit stabilisierend biegestarr in diesem Sinne ist eine Biegesteifigkeit gemeint, die eine gewisse Biegung zulassen kann, wie sie bei einer Schiene einer Orthese üblich ist, jedoch jedenfalls derart gering ist, dass die stützende Funktion und Stabilisierung der Gliedmaße erfüllt ist.

Neben der Stabilisierungsrichtung kann die Wabenstruktur eine in der flächigen Erstreckung liegende Anpassungsrichtung aufweisen. Quer zu der Anpassungsrichtung ist die Wabenstruktur lokal biegsam ausgebildet. Dabei ist die Wabenstruktur quer zur Anpassungsrichtung derart biegsam ausgebildet, dass sie durch Biegung quer zur Anpassungsrichtung an die Kontur einer Gliedmaße eines Patienten der Orthese in flächige Anlage bringbar ist. Die hierfür nötige Biegung kann beim Anlegen manuell erzielt werden. Bspw. kann die Orthese bzw. deren Wabenstruktur durch entsprechende Befestigungsgurte fixiert und gleichzeitig durch Biegung an die lokale Körperform des Trägers angepasst werden. Die Befestigung und Anpassung mittels der Befestigungsgurte tragen auch zum festen Sitz der Spange bei. Quer zur Anpassungsrichtung ist die Wabenstruktur zwar biegsam, sie bietet einer Biegung jedoch insbesondere einen gewissen Widerstand, sodass sie eine Spannung beim Anlegen aufrechterhält. Die im Sinne dieser Erfindung diskutierten Biegungen der Wabenstrukturen beziehen sich auf eine Biegung in Richtung der lokalen Flächennormalen der Erstreckung der Wabenstruktur.

Insbesondere kann durch eine gezielte Auswahl der Platzierung und Dimensionierung einzelner Stege und eine Variation der Anzahl der Stege vorteilhaft eine lokale Einstellung der Steifigkeit der Strukturkomponente über ihre flächige Erstreckung erreicht werden. Hierdurch können gezielt einzelne Bereiche der Wabenstruktur biegesteif und andere Bereiche flexibel gestaltet werden. Verschiedene lokale Steifigkeiten und Flexibilitätsgrade sind einfach realisierbar. Die Wabenstruktur weist insbesondere einen geschlossenen Rand auf. Es enden also keine Stege in Richtung ihrer Längserstreckung im Rand. Der Rand wird vielmehr durch einen umlaufenden Steg gebildet. Die materialfreien Aussparungen weisen insbesondere eine Vielzahl unterschiedlicher Formen auf, insbesondere wenigstens 3, insbesondere 5, insbesondere 7, verschiedene Formen an Aussparungen an einer Wabenstruktur. Insbesondere sind die Aussparungen überwiegend oder ausschließlich mit verrundeten Ecken ausgebildet. Die Stecke können sich in Richtung von Verbindungspunkten mit weiteren Stegen in ihrer Dicke in der flächigen Erstreckung der Wabenstruktur verdicken. Insbesondere gibt in einer Wabenstruktur verschiedenartige Verbindungspunkte, insbesondere Verbindungspunkte, in denen ein Steg auf einen gerade weiterverlaufenden Steg trifft, Verbindungspunkte, in denen mehrere Stege auf einen gerade weiterverlaufenden Steg treffen und Verbindungspunkte in denen mehrere (insbesondere 3 bis 6, insbesondere bis 5) Stege aus unterschiedlichen Richtungen aufeinandertreffen. Die Wabenstruktur kann Verbindungspunkte umfassen, in denen sich 2 gerade weiterverlaufende Stege kreuzen. Die unterschiedlichen Verbindungspunkte können in unterschiedlichen Kombinationen in der Wabenstruktur vorhanden sein.

Ein vorteilhafter Aspekt der Erfindung sieht vor, dass die Wabenstruktur mit der wenigstens einen Ausnehmung derart ausgebildet ist, dass die Pelotte mit dem Schalenelement in einer ersten Orientierung und einer, insbesondere der ersten Orientierung entgegengesetzten, zweiten Orientierung verbindbar ist.

Ein vorteilhafter Aspekt der Erfindung sieht vor, dass die wenigstens eine Pelotte derart mit dem Schalenelement verbindbar ist, dass die Rippenachse im vorgesehenen Tragezustand zumindest im Wesentlichen parallel zu Muskelfasern der radialen und/oder ulnaren Unterarmmuskulatur des Patienten verläuft. Demnach kann eine optimale Tiefenwirkung der Mikromassage der Unterarmmuskulatur erzielt werden.

Ein vorteilhafter Aspekt der Erfindung sieht vor, dass die wenigstens eine Pelotte wenigstens eine senkrecht zur Rippenachse verlaufende Pelottennase zum Beaufschlagen von Muskelfasern hin zur wenigstens einen Rippe aufweist. Dies ist besonders vorteilhaft, da die Unterarmmuskulatur im Bereich der Pelottennase hin zur Rippe beaufschlagt werden kann, sodass die Rippe effektiver auf die Unterarmmuskulatur einwirken kann.

Ein vorteilhafter Aspekt der Erfindung sieht vor, dass die wenigstens eine Pelotte zwei Pelottennasen aufweist. Demnach kann die Beaufschlagungswirkung der Pelotte weiter verstärkt werden. Zudem ist vorzugsweise die wenigstens eine Rippe senkrecht zur Rippenachse außermittig zu den zwei Pelottennasen angeordnet. Vorzugsweise ist eine Pelottennase länger als die andere Pelottennase ausgebildet, sodass eine gerichtete Beaufschlagung der Unterarmmuskulatur hin zur Rippe in eine Richtung verstärkt werden kann.

Ein vorteilhafter Aspekt der Erfindung sieht vor, dass die wenigstens eine Ausnehmung der Wabenstruktur als Durchbruch durch das wenigstens eine Schalenelement ausgebildet ist.

Ein vorteilhafter Aspekt der Erfindung sieht vor, dass die Ellenbogenspange zwei zueinander separat, und insbesondere identisch, ausgebildete Schalenelemente aufweist. Demnach kann eine Einschnürung der Blutzirkulation und ein Einengen des Radial- bzw. Ulnarnervs weiter vermindert werden.

Ein vorteilhafter Aspekt der Erfindung sieht vor, dass das wenigstens eine Schalenelement wenigstens eine, insbesondere schlitzartige, erste Befestigungsaufnahme zur lösbaren oder nicht-lösbaren Aufnahme des wenigstens einen Bandelements aufweist. Demnach kann das Bandelement einfach angebracht werden.

Ein vorteilhafter Aspekt der Erfindung sieht vor, dass die erste Befestigungsaufnahme eine primäre Befestigungsaufnahme und eine sekundäre Befestigungsaufnahme aufweist, wobei die primäre Befestigungsaufnahme in einem Mittenbereich und/oder die sekundäre Befestigungsaufnahme in einem Randbereich des wenigstens einen Schalenelements angeordnet ist. Das Ende des Bandelements, welches in der primären Befestigungsaufnahme, als im Mittenbereich, angeordnet ist, wird vorzugsweise außen am Schalenelement entlang geführt. Demnach ist eine gezielte Umlenkung des Bandelements gewährleistet. Dies kann vorzugsweise für besonders sensible Bereich des Unterarms Verwendung finden.

Ein vorteilhafter Aspekt der Erfindung sieht vor, dass die wenigstens eine Pelotte steifer als das wenigstens eine Schalenelement und/oder das wenigstens eine Schalenelement steifer als das wenigstens eine Bandelement ausgebildet ist. Demnach kann eine gezielte Massagewirkung mit der Pelotte erzielt werden, wobei das Schalenelement der Ellenbogenspange eine Form gibt. Das Bandelement stellt eine einfache Befestigung des Schalenelements am Unterarm dar.

Ein vorteilhafter Aspekt der Erfindung sieht vor, dass das wenigstens eine Schalenelement wenigstens eine, vorzugsweise vier, insbesondere punktförmige, zweite Befestigungsaufnahme zur Aufnahme wenigstens eines Polsters aufweist. Die zweiten Befestigungsaufnahmen können ebenfalls eine Ausnehmung der Wabenstruktur sein, wobei insbesondere diese als Durchbruch oder als Sackloch ausgebildet ist. Demnach kann an dem wenigstens einen Schalenelement alternativ zueinander oder in Kombination einfach wenigstens eine Pelotte und wenigstens ein Polster angebracht werden. Die Ausnehmung für die wenigstens eine Pelotte ist vorzugsweise im Mittenbereich und/oder die Ausnehmung für das Polster ist vorzugsweise im Randbereich des Schalenelements angeordnet.

Ein vorteilhafter Aspekt der Erfindung sieht vor, dass die wenigstens eine Pelotte und/oder das wenigstens eine Polster wenigstens einen Befestigungsabschnitt zum Eingreifen in die wenigstens eine Ausnehmung und/oder in die wenigstens eine zweite Befestigungsaufnahme aufweist. Der wenigstens eine Befestigungsabschnitt kann als hervorstehender Stift ausgebildet sein, wobei der jeweilige Befestigungsabschnitt mit der jeweiligen Ausnehmung als formschlüssig zusammenwirkt. Die Pelotte und/oder das Polster sind vorzugsweise in das Schalenelement einclipsbar.

Ein vorteilhafter Aspekt der Erfindung sieht vor, dass die wenigstens eine Pelotte zwischen dem wenigstens einen Schalenelement und dem wenigstens einen Polster angeordnet ist. Demnach kann zur Anlage an den Unterarm eine homogene Oberfläche von der Ellenbogenspange bereitgestellt werden.

Ein vorteilhafter Aspekt der Erfindung sieht vor, dass die Ellenbogenspange derart zur Anordnung am Unterarm des Patienten ausgebildet ist, dass die wenigstens eine Pelotte an M. extensor carpi radialis brevis und/oder an M. flexore antebrach des Patienten anliegt. Demnach ist eine besonders gute Wirkung der Pelotte auf die Unterarmmuskulatur erzielbar.

Die Ellenbogenspange ist derart ausgebildet, dass das wenigstens eine Schalenelement, insbesondere zwei Schalenelemente, und das wenigstens eine Bandelement, insbesondere zwei Bandelemente, eine Unterarmaufnahme zur Aufnahme des Unterarms bilden.

Das wenigstens eine Schalenelement ist gebogen ausgebildet, wobei die Biegung des wenigstens einen Schalenelements geringer als die Biegung der ulnaren oder radialen Oberfläche des Unterarms. Demnach kommt das Schalenelement vorzugsweise nur im Bereich der Pelotte zur Anlage an den Unterarm. Demnach wird die Tiefenwirkung weiter verbessert.

Ein vorteilhafter Aspekt der Erfindung sieht vor, dass die Pelotte, insbesondere an deren Außenseite, eine die erste Konfiguration anzeigende erste Markierung, insbesondere ein erstes Symbol und/oder eine erste Geometrie und/oder eine erste Farbe, vorzugsweise den Buchstaben "T" für die Behandlung eines Tennisarms, und eine die zweite Konfiguration anzeigende zweite Markierung, insbesondere ein zweites Symbol und/oder eine zweite Geometrie und/oder eine zweite Farbe, vorzugsweise den Buchstaben "G" für die Behandlung eines Golferarms, aufweist. Die Pelotte ist vorzugsweise derart am Schalenelement angeordnet, dass an einer Außenseite des Schalenelements je nach Konfiguration entweder die erste Markierung oder die zweite Markierung sichtbar ist. Die erste Markierung bzw. die zweite Markierung sind vorzugsweise durch die Ausnehmung des Schalenelements, insbesondere die Ausnehmung der Wabenstruktur, ersichtlich. Folglich ist von außen, auch im Tragezustand, einfach ersichtlich, in welcher Konfiguration die Pelotte eingerichtet ist.

Die der Erfindung zugrundeliegende Aufgabe wird ebenfalls gelöst durch eine Pelotte mit den Merkmalen des Anspruchs 14. Die Erfindung ist gerichtet auf eine Pelotte für eine Ellenbogenspange, die Pelotte umfassend wenigstens eine an einer Innenseite der Pelotte angeordnete, sich entlang einer Rippenachse erstreckende Rippe, wobei die Pelotte an der Innenseite senkrecht zur Rippenachse asymmetrisch ausgebildet ist. Die Innenseite, insbesondere die Rippe, kommt im Tragezustand zur Anlage an den Patienten.

Es ist vorteilhaft, wenn die Pelotte an der Außenseite, insbesondere die Befestigungsabschnitte der Pelotte, symmetrisch und die wenigstens eine Rippe asymmetrisch bzw. außermittig, insbesondere relativ zu den Befestigungsabschnitten, ausgebildet sind. Demnach kann die Pelotte in zwei Pelottenkonfigurationen an dem Schalenelement angeordnet werden, wobei die Pelotte als Ganzes grundsätzlich an derselben Position am Schalenelement angeordnet ist und nur die Position der wenigstens einen Rippe senkrecht zur Rippenachse verändert ist. Da die radiale Unterarmmuskulatur ("Tennisarm") höher als die ulnare Unterarmmuskulatur ("Golferarm") angeordnet ist, kann demnach mit einer einzigen Pelotte in beiden Konfigurationen entweder die radiale Unterarmmuskulatur oder die ulnare Unterarmmuskulatur behandelt werden.

Ein vorteilhafter Aspekt der Erfindung sieht vor, dass die Pelotte wenigstens eine senkrecht zur Rippenachse erstreckende, insbesondere zwei, Pelottennase aufweist. Die wenigstens eine Rippe ist vorzugsweise in Bezug auf die zwei Pelottennasen außermittig angeordnet.

Die Pelotte ist an eine zuvor beschriebene Ellenbogenspange, insbesondere an die Wabenstruktur des Schalenelements, anordenbar. Die Rippe erzielt eine besonders gute Massagewirkung der Unterarmmuskulatur im an der Ellenbogenspange angeordneten Zustand (Tragezustand). Mittels der Pelottennase kann die Unterarmmuskulatur in Richtung der Rippe geführt werden.

Ein vorteilhafter Aspekt der Erfindung sieht vor, dass die Pelotte zwei einander gegenüberliegende Pelottennasen aufweist, und insbesondere wobei die wenigstens eine Rippe in Bezug auf die Pelottennasen außermittig angeordnet ist. Demnach kann eine effektive und gezielte Führung der Unterarmmuskulatur in Richtung der Rippe erzielt werden.

Ein vorteilhafter Aspekt der Erfindung sieht vor, dass eine erste Nasenlänge der ersten Pelottennase und eine zweite Nasenlänge der zweiten Pelottennase unterschiedlich ausgebildet sind.

Ein vorteilhafter Aspekt der Erfindung sieht vor, dass die Pelotte hin zur Innenseite gebogen ausgebildet ist. Demnach kann sich die Pelotte besonders gut an den Unterarm anschmiegen.

Ein vorteilhafter Aspekt der Erfindung sieht vor, dass die Pelotte an einer der Innenseite gegenüberliegenden Außenseite wenigstens einen Befestigungsabschnitt zur Verbindung mit einem Schalenelement aufweist. Die Pelotte ist vorzugsweise in das Schalenelement einclipsbar. Demnach kann die Pelotte einfach an einem Schalenelement der Ellenbogenspange befestigt werden.

Die Pelotte ist vorzugsweise aus Kunststoff hergestellt. Die Pelotte weist vorzugsweise eine Shore-Härte in einem Bereich zwischen Shore A 5 und 50, insbesondere zwischen 10 und 30, auf.

Die der Erfindung zugrundeliegende Aufgabe wird ebenfalls gelöst durch eine Ellenbogenspange zur Entlastung einer radialen und/oder ulnaren Unterarmmuskulatur eines Patienten, umfassend: wenigstens ein Schalenelement mit einer Wabenstruktur, wenigstens ein Bandelement zum Befestigen des wenigstens einen Schalenelements an einem Unterarm des Patienten, und wenigstens eine mit dem Schalenelement und/oder der Wabenstruktur einstückig ausgebildete zuvor beschriebene Pelotte. Aufgrund der einstückigen Ausbildung des Schalenelements und der Pelotte kann die Ellenbogenspange besonders einfach am Patienten angeordnet werden.

Weitere Vorteile, Merkmale und Einzelheiten ergeben sich aus der nachfolgenden Beschreibung, in der unter Bezugnahme auf die Zeichnung verschiedene Ausführungsbeispiele der Erfindung dargestellt sind. Dabei können die in den Ansprüchen und der Beschreibung genannten Merkmale jeweils einzeln für sich oder in beliebiger Kombination erfindungswesentlich sein.

Es zeigen:
- Fig. 1: eine perspektivische Ansicht einer Ellenbogenspange;
- Fig. 2: eine weitere perspektivische Ansicht einer Ellenbogenspange;
- Fig. 3: eine perspektivische Ansicht eines Schalenelements mit einer Pelotte;
- Fig. 4A: eine Vorderansicht eines Schalenelements mit einer Pelotte in einer ersten Orientierung;
- Fig. 4B: eine Vorderansicht eines Schalenelements mit einer Pelotte in einer zweiten Orientierung;
- Fig. 5: eine Ansicht einer Pelotte;
- Fig. 6, 6A: Ansichten einer Pelotte;
- Fig. 7: eine perspektivische Ansicht eines Polsters;
- Fig. 8: eine Schnittansicht eines Polsters und zweier Schalenelemente einer Ellenbogenspange;
- Fig. 9: eine Seitenansicht der Ellenbogenspange mit der Pelotte in der ersten Konfiguration zur Behandlung eines Tennisarms; und
- Fig. 10: eine Seitenansicht der Ellenbogenspange mit der Pelotte in der zweiten Konfiguration zur Behandlung eines Golferarms.

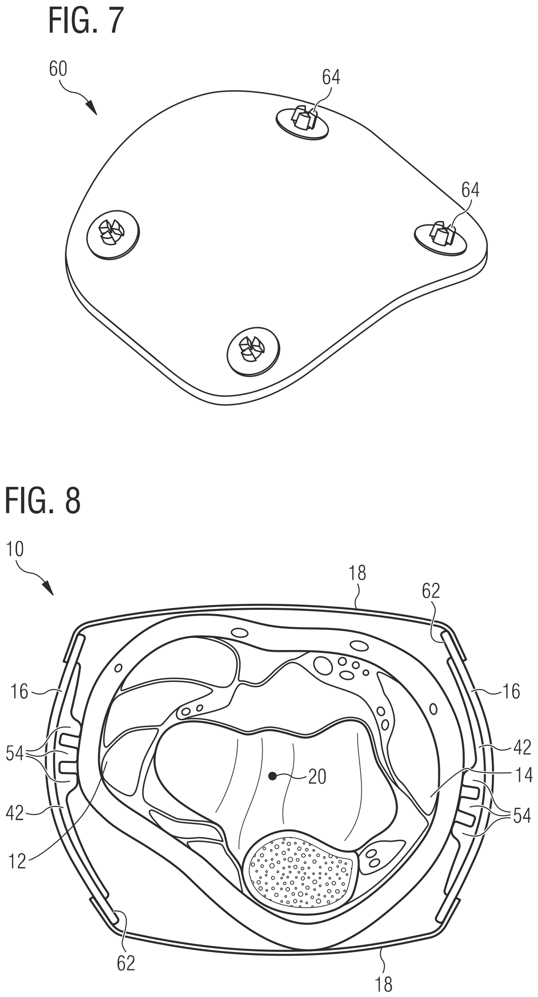

Die Ellenbogenspange 10 ist gemäß Fig. 8 zur Entlastung der radialen Unterarmmuskulatur 12 eines Patienten und der ulnaren Unterarmmuskulatur 14 eines Patienten ausgebildet, indem diese massiert bzw. wiederholend mit einer Kraft beaufschlagt werden.

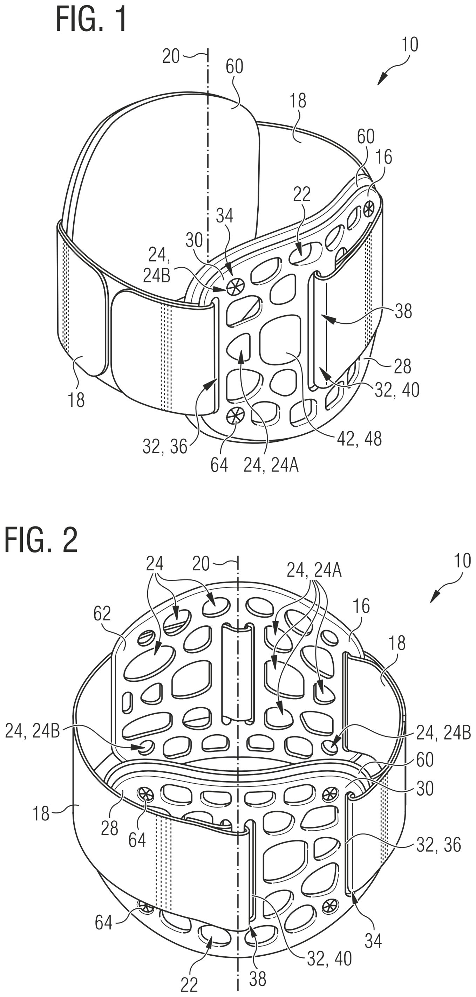

Die Ellenbogenspange 10 weist gemäß Fig. 1, 2 und 8 zwei Schalenelemente 16 sowie zwei die Schalenelemente 16 verbindende Bandelemente 18 auf. Die Ellenbogenspange 10 ist demnach hohlzylindrisch ausgebildet. Mittels zumindest eines der beiden Bandelemente 18 ist die Ellenbogenspange 10 in ihrer Spangenweite einstellbar, sodass diese für unterschiedliche Unterarmdurchmesser verwendbar ist. Dazu kann vorzugsweise ein Klettverschluss Verwendung finden. Im angelegten Zustand liegt die Ellenbogenspange 10 um den Unterarm des Patienten herum an, wobei eines der beiden Schalenelemente 16 im Bereich der radialen Unterarmmuskulatur 12 und ein weiteres der beiden Schalenelemente 16 im Bereich der ulnaren Unterarmmuskulatur 14 angeordnet ist. Im angelegten Zustand erstreckt sich der Unterarm entlang einer Längsachse 20 der Ellenbogenspange 10, wobei die Längsachse 20 im angelegten Zustand der Ellenbogenspange 10 im Wesentlichen parallel zu den Muskelfasern der radialen Unterarmmuskulatur 12 und/oder der ulnaren Unterarmmuskulatur 14 verläuft.

Die Schalenelemente 16 sind gemäß Fig. 8 im angelegten Zustand zur Längsachse 20 hin gekrümmt ausgebildet. Die Schalenelemente 16 weisen vorzugsweise gemäß Fig. 1 bis 4B jeweils eine Wabenstruktur 22 auf, welche eine Vielzahl von Ausnehmungen 24 in Form von Durchbrüchen aufweist. Die Ausnehmungen 24 sind in Bezug einer Spiegelachse 26 zumindest im Wesentlichen spiegelsymmetrisch verteilt. An einer ersten Schalenseite 28 des Schalenelements 16 sind größere Ausnehmungen 24 vorgesehen als einer der ersten Schalenseite 28 senkrecht zur Spiegelachse 26 gegenüberliegenden zweiten Schalenseite 30 des Schalenelements 16.

Zur Befestigung der Bandelemente 18 weist das Schalenelement 16 erste Befestigungsaufnahmen 32, insbesondere in einem Randbereich 34 des Schalenelements 16 einen ersten Schlitz 36 und in einem Mittenbereich 38 des Schalenelements 16 zwei zweite Schlitze 40, auf, wobei die Bandelemente 18 jeweils mit einer Schlaufe durch den ersten Schlitz 36 und die beiden zweiten Schlitze 40 durchgeführt werden. Der erste Schlitz 36 ist vorzugsweise an der zweiten Schalenseite 30 angeordnet. Aufgrund der Anordnung eines Bandelements 18 am Mittenbereich 38 des Schalenelements 16 wird das Bandelement 18 parallel zur Oberfläche des Unterarms geführt, was ein schonenderes Tragen der Ellenbogenspange 10 mit sich bringt. Der erste Schlitz 36 und/oder die zweiten Schlitze 40 verlaufen vorzugsweise parallel zur Längsachse 20.

Die Ellenbogenspange 10 weist ferner gemäß Fig. 1, 3 bis 4B und 8 eine Pelotte 42 auf, welche lösbar an dem Schalenelement 16 befestigbar ist. Die Pelotte 42 ist mit pelottenzugehörigen Ausnehmungen 24A in der Wabenstruktur 22 lösbar formschlüssig verbunden. Die pelottenzugehörigen Ausnehmungen 24A für die Pelotte 42 weisen eine im Wesentlichen quadratische Grundform mit abgerundeten Ecken auf. Dabei sind jeweils vier pelottenzugehörige Ausnehmungen 24A, insbesondere mittig, rechts, links und hinten, je Seite, also erster Schalenseite 28 und zweiter Schalenseite 30, angeordnet. Die pelottenzugehörigen Ausnehmungen 24A sind dabei vorzugsweise T-förmig angeordnet. Die mittlere pelottenzugehörigen Ausnehmung 24A ist vorzugsweise größer als die weiteren pelottenzugehörigen Ausnehmungen 24A ausgebildet.

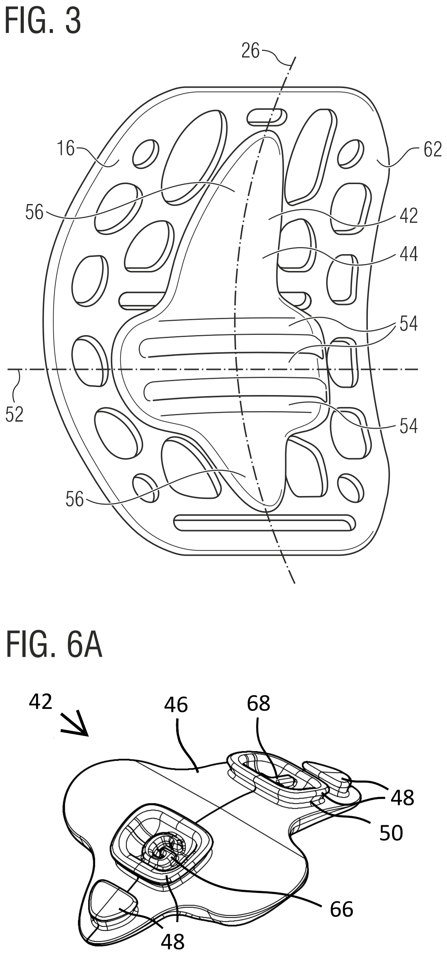

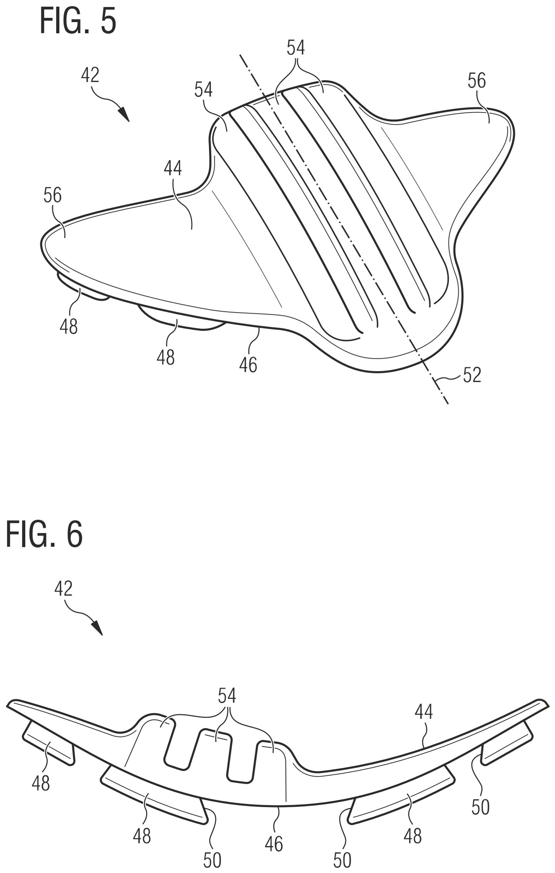

Die Pelotte 42 weist eine Innenseite 44 und eine Außenseite 46 auf. Die Pelotte 42 weist gemäß Fig. 6 und Fig. 6A an der Außenseite 46 jeweils zu den pelottenzugehörigen Ausnehmungen 24A korrespondierende Befestigungsabschnitte 48 mit Hinterschnitten 50 auf, wobei die Befestigungsabschnitte 48 in die pelottenzugehörigen Ausnehmungen 24A eingreifen und die Hinterschnitte 50 das Schalenelement 16 bzw. die Wabenstruktur 22 hintergreifen. Vorzugsweise sind die mittleren und hinteren Befestigungsabschnitte 48 vollständig und die linken und rechten Befestigungsabschnitte 48 nur innenseitig zu den pelottenzugehörigen Ausnehmungen 24A korrespondierend ausgebildet. Die Befestigungsabschnitte 48 sind leicht flexible ausgebildet, sodass die Befestigungsabschnitte 48 leicht in die Ausnehmungen 24 einführbar sind. Damit ist ein sicherer Halt der Pelotte 42 an dem Schalenelement 16 gewährleistet.

Alternativ können gemäß Fig. 6A auch nur zwei mittlere und zwei hintere Befestigungsabschnitte 48 vorgesehen sein.

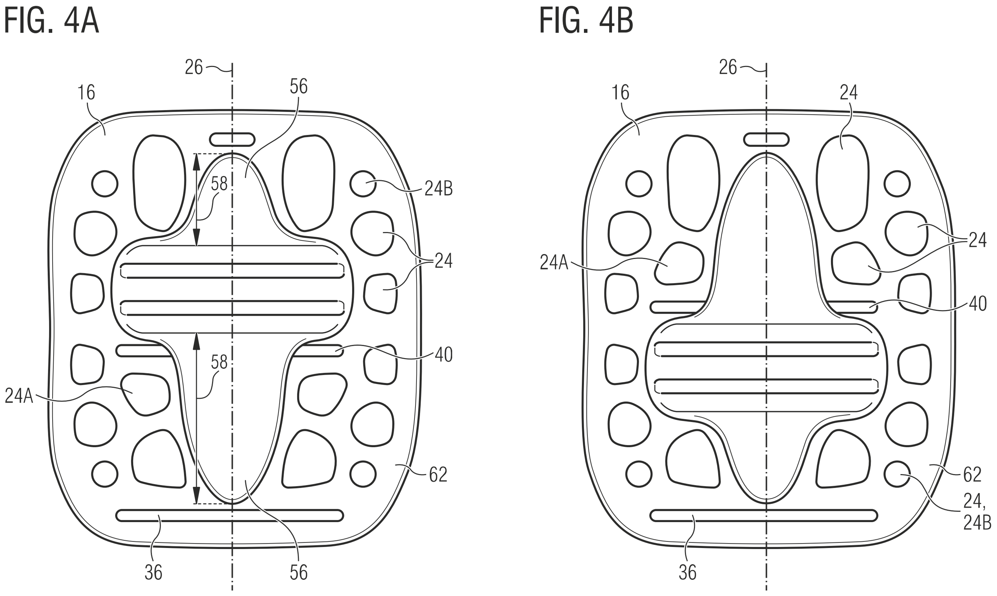

Die Pelotte 42 kann gemäß der Fig. 4A und 4B in zwei verschiedenen Orientierungen an dem Schalenelement 16 angeordnet werden. Dazu sind die Befestigungsabschnitte 48 vorzugsweise symmetrisch ausgebildet. Die Pelotte 42 weist gemäß Fig. 3 bis 5 an der Innenseite 44 parallel zu einer Rippenachse 52, welche vorzugsweise senkrecht zur Spiegelachse 26 verläuft, sich erstreckende Rippen 54. Die Rippen 54 dienen für eine besonders effektive und zielgerichtete Massagewirkung der Unterarmmuskulatur 12. Die Pelotte 42 ist dabei derart am Schalenelement 16 angeordnet, dass die Rippenachse 52 im vorgesehenen Tragezustand zumindest im Wesentlichen parallel zu Muskelfasern der radialen Unterarmmuskulatur 12 und/oder ulnaren Unterarmmuskulatur 14 des Patienten verläuft. Demnach kann eine optimale Tiefenwirkung der Mikromassage der Unterarmmuskulatur 12 erzielt werden. Aufgrund der unterschiedlichen Orientierungen der Pelotte 42 kann diese entweder an der radialen Unterarmmuskulatur 12 oder an der ulnaren Unterarmmuskulatur 14 des Patienten angewendet werden. Dazu sind die Rippen 54 gegenüber der Befestigungsabschnitte 48 außermittig angeordnet. Dies zeigt sich insbesondere dadurch, dass die Pelotte 42 zwei einander gegenüberliegende, senkrecht zur Rippenachse 52 sich erstreckende Pelottennasen 56 aufweist, welche eine unterschiedliche Nasenlänge 58 aufweisen. Demnach ist die Innenseite 44 der Pelotte 42 hinsichtlich der Rippen 54 asymmetrisch und die Außenseite 46 der Pelotte 42 hinsichtlich der Befestigungsabschnitte 48 symmetrisch ausgebildet.

Die Pelottennase 56 sind vorzugsweise derart ausgebildet, dass diese die Muskelfasern hin zu den Rippen 54 mit einer Kraft beaufschlagen, sodass die Muskelfasern zu den Rippen 54 zumindest leicht gedrängt werden.

Die erste Befestigungsaufnahme 32 erstreckt sich senkrecht zur Spiegelachse 26. Die erste Befestigungsaufnahme 32 ist gemäß Fig. 2 vorzugsweise zwischen den vier pelottenzugehörigen Ausnehmungen 24A der ersten Schalenseite 28 und der zweiten Schalenseite 30 angeordnet. Demnach sind die pelottenzugehörigen Ausnehmungen 24A auch symmetrisch zum Mittenbereich 38 angeordnet.

Entlang der Spiegelachse 26 ist das Schalenelement 16 länger als die Pelotte 42 ausgebildet.

Ferner weist die Ellenbogenspange 10 gemäß Fig. 2 ein Polster 60 oder gemäß Fig. 1 zwei Polster 60 zur Anordnung an einer Schaleninnenseite 62 des Schalenelements 16. Das Polster 60 kann unmittelbar an das Schalenelement 16 angeordnet werden, wie in Fig. 2 gezeigt. Alternativ kann zwischen dem Schalenelement 16 und dem Polster 60 eine Pelotte 42 vorgesehen sein, wie in Fig. 1 gezeigt. Dazu weist die Wabenstruktur 22 im Randbereich 34 vier, insbesondere punktförmige, polsterzugehörige Ausnehmungen 24B auf, in welche Polsterbefestigungsabschnitte 64 eingreifen können. Die Polsterbefestigungsabschnitte 64 können ebenfalls Hinterschnitte 50 aufweisen. Das Polster 60 ist deckt das Schalenelement 16 vorzugsweise vollständig ab, sodass die Haut des Patienten nur durch das Polster 60 kontaktiert wird.

Das Schalenelement 16 ist vorzugsweise hin zur Schaleninnenseite 62 bzw. zur Längsachse 20 gebogen ausgebildet. Die Pelotte 42 ist vorzugsweise zur Innenseite 44 bzw. zur Längsachse 20 gebogen ausgebildet. Das Polster 60 ist flach ausgebildet und ist im Tragezustand hin zu Längsachse 20 gebogen ausgebildet.

Die Ellenbogenspange 10 ist in Fig. 8 im Tragezustand, also im am Unterarm des Patienten angeordneten Zustand, gezeigt. Die Ellenbogenspange 10 weist gemäß Fig. 8 zwei identische Schalenelemente 16 auf. Zur Behandlung der radialen Unterarmmuskulatur 12 ist die Pelotte 42 in der ersten Orientierung gemäß Fig. 4A am Schalenelement 16 angeordnet und damit höher gelegen. Zur Behandlung der ulnaren Unterarmmuskulatur 14 ist die Pelotte 42 in der zweiten Orientierung gemäß Fig. 4B am Schalenelement 16 angeordnet und damit niedriger gelegen. Es ist denkbar, dass zwei Schalenelemente 16 und eine Pelotte 42 zum Einsatz kommen. Es ist ebenfalls denkbar, dass zwei Schalenelement 16 mit jeweils einer Pelotte 42 zum Einsatz kommen. Die beiden Schalenelement 16 werden oberseitig und unterseitig jeweils durch ein Bandelement 18 miteinander verbunden. Demnach kann die Ellenbogenspange 10 mittels der Bandelemente 18 auf die Größe des Unterarms angepasst werden. Aufgrund der sich aus der Wabenstruktur 22 ergebenden Flexibilität der Schalenelemente 16 schmiegen diese sich durch den Zug der Bandelement 18 an den Unterarm an.

Im Tragezustand liegt die die Ellenbogenspange 10 vorzugsweise derart am Unterarm des Patienten an, dass die wenigstens eine Pelotte 42 an M. extensor carpi radialis brevis und/oder an M. flexore antebrach des Patienten anliegt.

Es ist ebenfalls denkbar, dass die Schalenelemente 16 und die Pelotte 42 und/oder das Polster 60 nicht voneinander lösbar ausgebildet sein. Die Ellenbogenspange 10 kann dabei ein- oder mehrteilig ausgebildet sein.

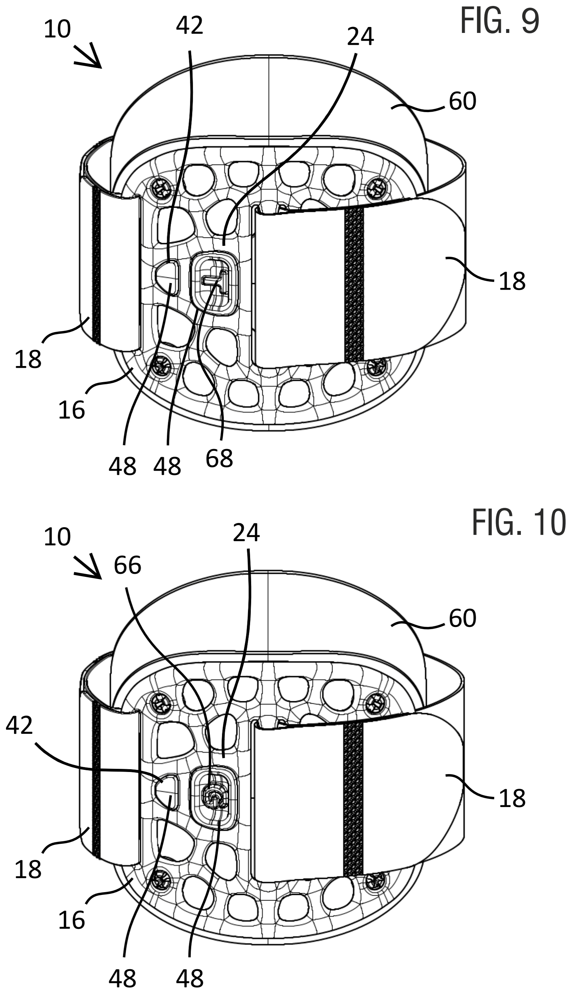

Gemäß Fig. 9 und 10 weist die Pelotte 42 an der Außenseite 46 eine erste Markierung 66 in Form eines erhabenen Buchstabens "G" und eine zweite Markierung 68 in Form eines erhabenen Buchstabens "T" auf. Die Pelotte 42 ist gemäß Fig. 9 derart an dem Schalenelement 16 anordenbar, dass in der ersten Konfiguration bzw. in der ersten Pelottenorientierung nur die erste Markierung 66 von außen durch das Schalenelement 16 sichtbar ist ("Tennisarm"). Die zweite Markierung 68 wird in der zweiten Konfiguration durch das Bandelement 18 und/oder das Schalenelement 16 abgedeckt. Die Pelotte 42 ist derart an dem Schalenelement 16 anordenbar, dass in der zweiten Konfiguration bzw. in der zweiten Pelottenorientierung nur die zweite Markierung 68 von außen durch das Schalenelement 16 sichtbar ist ("Golferarm"). Die erste Markierung 66 wird in der zweiten Konfiguration durch das Bandelement 18 und/oder das Schalenelement 16 abgedeckt. Obwohl die Pelotte 42 durch das Schalenelement 16 im Wesentlichen abgedeckt ist, kann von außen eingesehen werden, in welcher Konfiguration die Pelotte 42 eingerichtet ist. Vorzugsweise ist die erste Markierung 66 und/oder die zweite Markierung 68 an oder in einem Befestigungsabschnitt 48 angeordnet. Wenn eine erste Markierung 66 und/oder eine zweite Markierung 68 an der Pelotte 42 vorgesehen ist, kann der Befestigungsabschnitt 48 hohl ausgebildet sein, sodass die erste Markierung 66 und/oder die zweite Markierung 68 nicht gegenüber dem Befestigungsabschnitt 48 hervorsteht.

## Patentansprüche

1. Ellenbogenspange (10) zur Entlastung einer radialen Unterarmmuskulatur (12) und/oder einer ulnaren Unterarmmuskulatur (14) eines Patienten, umfassend:
- wenigstens ein Schalenelement (16) zur medialen und/oder lateralen Anordnung an den Unterarm des Patienten;
- wenigstens ein Bandelement (18) zum Befestigen des wenigstens einen Schalenelements (16) an dem Unterarm des Patienten;
- wenigstens eine am wenigstens einen Schalenelement (16) definiert angeordnete Pelotte (42) mit wenigstens einer sich entlang einer Rippenachse (52) erstreckenden Rippe (54), wobei die Pelotte (42) in einer definierten ersten Konfiguration zur Behandlung der radialen Unterarmmuskulatur (12) und in einer definierten zweiten Konfiguration zur Behandlung der ulnaren Unterarmmuskulatur (14) einrichtbar ist.

2. Ellenbogenspange (10) nach Anspruch 1, wobei das wenigstens eine Schalenelement (16) und die wenigstens eine Pelotte (42) lösbar oder unlösbar miteinander verbunden sind.

3. Ellenbogenspange (10) nach einem der Ansprüche 1 oder 2, wobei das wenigstens eine Schalenelement (16) wenigstens eine Ausnehmung (24) und/oder die wenigstens eine Pelotte (42) wenigstens einen Befestigungsabschnitt (48) zum formschlüssigen und lösbaren Verbinden des wenigstens einen Schalenelements (16) und der wenigstens einen Pelotte (42) aufweist.

4. Ellenbogenspange (10) nach einem der Ansprüche 1 bis 3, wobei die wenigstens Pelotte (42) senkrecht zur Rippenachse (52) asymmetrisch ausgebildet ist, insbesondere wobei die wenigstens eine Rippe (54) außermittig gegenüber einer senkrecht zur Rippenachse (52) verlaufenden Quererstreckung der wenigstens einen Rippe (54) ausgebildet ist.

5. Ellenbogenspange (10) nach einem der vorherigen Ansprüche, wobei die wenigstens eine Pelotte (42) in der ersten Konfiguration in einer ersten Pelottenorientierung an dem wenigstens einen Schalenelement (16) angeordnet ist, wobei die wenigstens eine Pelotte (42) in der zweiten Konfiguration in einer zweiten Pelottenorientierung an dem wenigstens einen Schalenelement (16) angeordnet ist, und wobei die erste Pelottenorientierung und die zweite Pelottenorientierung invertiert zueinander ausgebildet sind.

6. Ellenbogenspange (10) nach einem der vorherigen Ansprüche, wobei das wenigstens eine Schalenelement (16) in der ersten Konfiguration der wenigstens einen Pelotte (42) in einer ersten Schalenorientierung an dem wenigstens einen Bandelement (18) und/oder am Unterarm des Patienten angeordnet ist, wobei das wenigstens eine Schalenelement (16) in der zweiten Konfiguration der wenigstens einen Pelotte (42) in einer zweiten Schalenorientierung an dem wenigstens einen Bandelement (18) und/oder am Unterarm des Patienten angeordnet ist, und wobei die erste Schalenorientierung und die zweite Schalenorientierung invertiert zueinander ausgebildet sind.

7. Ellenbogenspange (10) nach einem der vorherigen Ansprüche, wobei die Ellenbogenspange (10) ein erstes Schalenelement (16) und ein zweites Schalenelement (16) aufweist, insbesondere wobei die Schalenelemente (16) an zwei gegenüberliegenden Enden über jeweils zwei Bandelemente (18) miteinander verbunden sind, insbesondere wobei ein erstes Bandelement (18) zur Festlegung des Abstands der Schalenelemente (16) und/oder das zweite Bandelement (18) dem Anlegen der Ellenbogenspange (10) dient, wobei in der ersten Konfiguration die Pelotte (42) zur Behandlung der radialen Unterarmmuskulatur (12) an dem ersten Schalenelement (16), insbesondere in der ersten Pelottenorientierung, und in der zweiten Konfiguration zur Behandlung der ulnaren Unterarmmuskulatur (14) an dem zweiten Schalenelement (16), insbesondere in der zweiten Pelottenorientierung, anordenbar oder angeordnet ist, insbesondere wobei die Schalenelemente (16) in einer gleichen Schalenorientierung angeordnet sind.

8. Ellenbogenspange (10) nach einem der vorherigen Ansprüche, wobei wenigstens eine erste Ausnehmung (24) und wenigstens eine zweite Ausnehmung (24) im wenigstens einen Schalenelement (16) vorgesehen sind, und wobei die wenigstens eine Pelotte (42) in der ersten Konfiguration an der wenigstens einen ersten Ausnehmung (24) und in der zweiten Konfiguration an der wenigstens einen zweiten Ausnehmung (24) angeordnet ist.

9. Ellenbogenspange (10) nach einem der vorherigen Ansprüche, wobei das wenigstens eine Schalenelement (16) eine Wabenstruktur (22) aufweist, und insbesondere wobei die wenigstens eine Ausnehmung (24) Teil der Wabenstruktur (22) ist.

10. Ellenbogenspange (10) nach einem der vorherigen Ansprüche, wobei das wenigstens eine Schalenelement (16) wenigstens eine, insbesondere punktförmige, zweite Befestigungsaufnahme (32) zur Aufnahme wenigstens eines Polsters (60) aufweist, und insbesondere wobei die wenigstens eine Pelotte (42) zwischen dem wenigstens einen Schalenelement (16) und dem wenigstens einen Polster (60) angeordnet ist.

11. Ellenbogenspange (10) nach einem der vorherigen Ansprüche, wobei die Ellenbogenspange (10), insbesondere die Pelotte (42), eine die Behandlung der ulnaren Unterarmmuskulatur (14) charakterisierende erste Markierung (66) und eine die Behandlung der radialen Unterarmmuskulatur (12) charakterisierende zweite Markierung (68) aufweist, wobei in der ersten Konfiguration der wenigstens einen Pelotte (42), insbesondere nur, die erste Markierung (66) und in der zweiten Konfiguration der wenigstens einen Pelotte (42), insbesondere nur, die zweite Markierung (68) sichtbar ist.

12. Pelotte (42) für eine Ellenbogenspange (10), die Pelotte (42) umfassend wenigstens eine an einer Innenseite (44) der Pelotte (42) angeordnete, sich entlang einer Rippenachse (52) erstreckende Rippe (54), wobei die Pelotte (42) an der Innenseite (44) senkrecht zur Rippenachse (52) asymmetrisch ausgebildet ist.

13. Pelotte (42) nach Anspruch 12, wobei die Pelotte (42) zwei sich senkrecht zur Rippenachse (52) erstreckende, einander gegenüberliegende Pelottennasen (56) aufweist, und insbesondere wobei die wenigstens eine Rippe (54) in Bezug auf die zwei Pelottennasen (56) außermittig angeordnet ist.

14. Pelotte (42) nach einem der Ansprüche 12 bis 13, wobei die Pelotte (42) an einer der Innenseite (44) gegenüberliegenden Außenseite (46) wenigstens einen Befestigungsabschnitt (48) zur Verbindung mit einem Schalenelement (16) aufweist.

15. Pelotte (42) nach einem der Anspruch 12 bis 13, wobei die Pelotte (42) an der Außenseite (46) eine die erste Konfiguration anzeigende erste Markierung (66) und eine die zweite Konfiguration anzeigende zweite Markierung (68) aufweist.
